# EUROPEAN PATENT SPECIFICATION

(11) **EP 3 802 483 B1**
(45) Date of publication and mention of the grant of the patent: **19.02.2025**
(21) Application number: 19737897.9
(22) Date of filing: 31.05.2019
(51) Int. Cl.: C07C 255/55, H10K 85/60

(54) **CYANO-DERIVATIZED OLIGONOPHENYLACETYLENE-BASED FLUORESCENT SMALL MOLECULES SUITABLE FOR USE IN ORGANIC LIGHT-EMITTING DIODE APPLICATIONS AND THEIR USE IN ORGANIC LIGHT-EMITTING DIODE APPLICATIONS**
FLUORESZIERENDE KLEINE MOLEKÜLE AUF DER BASIS VON CYANO-DERIVATISIERTEM OLIGONOPHENYLACETYLEN ZUR VERWENDUNG FÜR ORGANISCHE LICHTEMITTIERENDE DIODEN UND IHRE VERWENDUNG IN ORGANISCHEN LICHTEMITTIERENDEN DIODEN
PETITES MOLÉCULES FLUORESCENTES À BASE D'OLIGONOPHÉNYLACÉTYLÈNE CYANO-DÉRIVATISÉES APPROPRIÉES POUR UNE UTILISATION DANS DES APPLICATIONS DE DIODES ÉLECTROLUMINESCENTES ORGANIQUES ET LEUR UTILISATION DANS DES APPLICATIONS DE DIODES ÉLECTROLUMINESCENTES ORGANIQUES

(30) Priority: 05.06.2018 TR 201807984
(43) Date of publication of application: 14.04.2021
(73) Proprietor: Tubitak, 06100 Ankara (TR)
(72) Inventor: DABAK, Salih, 41470 Kocaeli (TR); USTA, Hakan, 38080 Kayseri (TR); TEKIN, Emine, 41470 Kocaeli (TR); ALIMLI EYIGÜN, Dilek, 41470 Kocaeli (TR); UÇAR, Esin, 06750 Ankara (TR); ÜLKÜ, Alper, 06750 Ankara (TR)
(86) International application number: PCT/IB2019/054513
(87) International publication number: WO 2019/234563

(56) References cited:
- JP-A- 2009 209 073
- NOELIA FUENTES ET AL: "On/off electrochemical switches based on quinone-bisketals", CHEMICAL COMMUNICATIONS, vol. 47, no. 5, 1 January 2011 (2011-01-01), pages 1586 - 1588, XP055609916, ISSN: 1359-7345, DOI: 10.1039/C0CC04388E
- TAKANORI OCHI ET AL: "Rod-shaped Oligophenyleneethynylenes Modified by Donor and Acceptor Groups in a Block Manner: Synthesis and Light-emitting Characteristics", CHEMISTRY LETTERS, vol. 36, no. 6, 5 June 2007 (2007-06-05), JAPAN, pages 794 - 795, XP055609918, ISSN: 0366-7022, DOI: 10.1246/cl.2007.794
- YOSHIHIRO YAMAGUCHI ET AL: "Highly Emissive Whole Rainbow Fluorophores Consisting of 1,4-Bis(2-phenylethynyl)benzene Core Skeleton: Design, Synthesis, and Light-Emitting Characteristics", JOURNAL OF PHYSICAL CHEMISTRY. A, MOLECULES, SPECTROSCOPY,KINETICS, ENVIRONMENT AND GENERAL THEORY, vol. 119, no. 32, 30 July 2015 (2015-07-30), US, pages 8630 - 8642, XP055609935, ISSN: 1089-5639, DOI: 10.1021/acs.jpca.5b05077
- GRIGORAS MIRCEA ET AL: "Photophysical properties of isoelectronic oligomers with vinylene, imine, azine and ethynylene spacers bearing triphenylamine and carbazole end-groups", DYES AND PIGMENTS, ELSEVIER APPLIED SCIENCE PUBLISHERS. BARKING, GB, vol. 98, no. 1, 13 February 2013 (2013-02-13), pages 71 - 81, XP028524439, ISSN: 0143-7208, DOI: 10.1016/J.DYEPIG.2013.01.025
- BAI XINYAN ET AL: "Synthesis and characterization of unsymmetrical phenylene-ethynylene trimers and tetramers with different chromophores as blue-light-emitters", TETRAHEDRON LETTERS, ELSEVIER, AMSTERDAM, NL, vol. 54, no. 13, 8 February 2013 (2013-02-08), pages 1711 - 1713, XP028993893, ISSN: 0040-4039, DOI: 10.1016/J.TETLET.2013.01.076

## Description

### Technical Field

The present invention relates to the synthesis of oligonophenylacetylene-based fluorescent π-conjugated small molecules, which are derivatized with group or groups of cyano (-CN) and have the ability to emit light with very sharp emission peaks at different wavelengths and relates to their use in organic light emitting diodes (OLEDs) as light-emitting active layers.

### Prior Art

The design, synthesis, and characterization of chemically-stable and solution-processable fluorescent small molecules, which emission wavelengths could be tuned via facile structural tailoring and could display high optical and electrical characteristics, is very crucial to the development of high-performance organic light-emitting diode technologies. Small molecules are organic compounds formed by linking a limited number of elements such as carbon, hydrogen, oxygen, nitrogen and sulfur via strong chemical bonds and it has well-defined three-dimensional (3-D) boundaries with clearly defined size and mass. The fact that the fluorescent organic small molecules, which have light-emitting properties, could be processed from solutions could seriously reduce the production costs for various displays and lighting devices. Despite the development of numerous inorganic and organic based materials until today, there has been no studies on cyano(-CN)-derivatized oligonophenylacetylene-based fluorescent π-conjugated molecules use in organic light emitting diode applications as electroluminescent active materials in light emitting layer. There is an emerging need for the development of new materials and devices that could be produced with lower cost by processing from solution and could exhibit very sharp electroluminescence peaks and good luminescence characteristics in organic light-emitting diodes.

The Chinese patent with the document number CN102786438 in the known state of the art, states a method to produce cyano-derivatized anthracene derivative and reports the corresponding organic electroluminescence device using cyano-derivatized anthracene derivative as the light-emitting layer.

United States patent with the document number US2002006479 mentions a liquid-crystal composition comprising a phenylacetylene compound. It mentions the benefits of using the phenylacetylene compound in the preparation of liquid-crystal compositions for optical imaging and recording, materials; also for optical compensator, and polarizer applications.

### Brief Description of the Invention

The aim of the present invention is to realize the synthesis of new oligonophenylacetylene-based fluorescent π-conjugated small molecules, which are derivatized with group or groups of cyano (-CN) and have light-emitting characteristics with very sharp emission peaks at different wavelengths.

Another aim of this invention is to realize the preparation of thin-films of these oligonophenylacetylene-based fluorescent π-conjugated small molecules, which are derivatized with group or groups of cyano (-CN) from their solutions.

Another aim of the present invention is to realize the use of these prepared active small molecule-based thin-films (light-emitting) in organic light emitting diodes.

Another aim of this invention is to realize organic light-emitting diodes with high luminescence efficiency, brightness, and external quantum yield with the help of small molecules.

Another aim of this invention is to provide a method of placing oligonophenylacetylene-based fluorescent π-conjugated small molecules, which are derivatized with group or groups of cyano (-CN) in organic light-emitting diodes.

### Detailed Description of the Invention

For the achievement of purpose of this invention, "Cyano-Derivatized Oligonophenylacetylene-Based Fluorescent Small Molecules Suitable For Use In Organic Light-Emitting Diode Applications And Their Use In Organic Light-Emitting Diode Applications" are given in attached figures; from these figures:
Figure 1 illustrates a schematic view of the synthesis of the molecule 2,2'-((2,5-bis((2-ethylhexyl)oxy)-1,4-phenylene)bis(ethyne-2,1-diyl))dibenzonitrile (A).
Figure 2 illustrates a schematic view of the synthesis of the molecule 4,4"-((2,5-bis((2-ethylhexyl)oxy)-1,4-phenylene)bis(ethyne-2,1-diyl))bis(4'-(diphenylamino)-[1,1'-biphenyl]-3-carbonitrile) (B).
Figure 3 illustrates a schematic view of the synthesis of the molecule 4,4"-((2,5-bis((2-octyldodecyl)oxy)-1,4-phenylene)bis(ethyne-2,1-diyl))bis(4'-(diphenylamino)-[1,1'-biphenyl]-3-carbonitrile) (C).
Figure 4 illustrates a schematic view of the synthesis of the molecule N,N-diphenyl-4-(4,4,5,5- tetramethyl -1,3,2- dioxaborolan -2-yl) aniline (5).
Figure 5 shows absorbance and photoluminescence profiles of cyano-derivatized oligophenylacetylene molecules (A, B, and C) in dichloromethane solution.
Figure 6 illustrates configurations of the OLED devices in which the cyano-derivatized oligophenylacetylene molecules used as the light-emitting layer .
Figure 7 shows the electroluminescence profiles of the OLEDs in which cyano-derivatized oligophenylacetylene molecules (A, B, C) used as light-emitting layers.

The chemical formulations contained in the figures are individually numbered and the correspondences of these numbers are given below:
**1.** 1,4-dibromo-2,5-bis((2-ethylhexyl)oxy)benzene
**2.** ((2,5-bis((2-ethylhexyl)oxy)-1,4-phenylene)bis(ethyne-2,1-diyl))bis(trimethylsilane)
**3.** 1,4-bis((2-ethylhexyl)oxy)-2,5-diethynylbenzene
**4.** 6,6'-((2,5-bis((2-ethylhexyl)oxy)-1,4-phenylene)bis(ethyne-2,1-diyl))bis(3-bromobenzonitrile)
**5.** N,N-diphenyl-4-(4,4,5,5-tetramethyl-1,3,2-dioxaborolan-2-yl) aniline
**6.** 1,4-dibromo-2,5-bis((2-octyldodecyl)oxy)benzene
**7.** ((2,5-bis((2-octyldodecyl)oxy)-1,4-phenylene)bis(ethyne-2,1-diyl))bis(trimethylsilane)
**8.** 1,4-bis((2-octyldodecyl)oxy)-2,5-diethynylbenzene
**9.** 6,6'-((2,5-bis((2-octyldodecyl)oxy)-1,4-phenylene)bis(ethyne-2,1-diyl))bis(3-bromobenzonitrile)

**A.** 2,2'-((2,5-bis((2-ethylhexyl)oxy)-1,4-phenylene)bis(ethyne-2,1-diyl))dibenzonitrile
**B.** 4,4"-((2,5-bis((2-ethylhexyl)oxy)-1,4-phenylene)bis(ethyne-2,1-diyl))bis(4'-(diphenylamino)-[1,1'-biphenyl]-3-carbonitrile)
**C.** 4,4"-((2,5-bis((2-octyldodecyl)oxy)-1,4-phenylene)bis(ethyne-2,1-diyl))bis(4'-(diphenylamino)-[1,1'-biphenyl]-3-carbonitrile)
**D.** Glass Substrate
**E.** ITO (indium tin oxide)
**F.** PEDOT:PSS (poly(3,4-ethylenedioxythiophene):polystyrenesulfonate)
   **A, B, C - 1.** CBP small molecule (host material: small molecule)
   **A, B, C** - **2.** small molecule
**I.** TPBI (electron transfer material)
**J.** Ca (calcium)
**K.** Al (aluminum)
**L.** LiF (lithium fluoride)

The general formulation of cyano-derivatized oligonophenylacetylene small molecules is as follows:

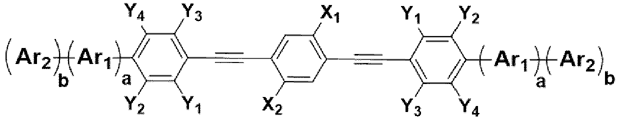

In a preferred application of the invention, X₁ and X₂ in the formulation of cyano-derivatized oligonophenylacetylene-based fluorescent small molecule is selected from a group with hydrogen atom independent of each other or straight chain containing 1 to 40 carbon atoms, branched saturated alkyl or alkoxy group, or straight chain having alkene or alkyne functional groups or branched unsaturated alkyl or alkoxy group.

In a preferred application of the invention, Y₁-Y₄ in the formulation of cyano-derivatized oligonophenylacetylene-based fluorescent small molecule is selected from a group with hydrogen atom or cyano (-CN) functional group or straight chain containing 1 to 40 carbons or branched saturated alkyl or alkoxy group or straight chain having alkene or alkyne functional groups or a group containing a branched unsaturated alkyl or alkoxy group provided that at least one of them is a cyano (-CN) functional group, independent from each other.

In a preferred application of the invention, Ar₁ in the formulation of cyano-derivatized oligonophenylacetylene-based fluorescent small molecule is selected from an aryl group of 6-20 carbon atoms with or without one or more alkyl or alkoxy substituents, and a heteroaryl group of 5-20 ring atoms with or without one or more alkyl or alkoxy substituents.

In one application of the invention, the formulation of Ar₂ in cyano-derivatized oligonophenylacetylene based fluorescent small molecule formulation is:

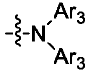

In the preferred application of the invention, Ar₃ in the Ar₂ formulation in the general formulation of the cyano-derivatized oligonophenylacetylene based fluorescent small molecule is selected from an aryl group of 6-20 carbon atoms with or without one or more alkyl or alkoxy substituents and a heteroaryl group of 5-20 atoms with or without one or more alkyl or alkoxy substituents.

In another application of the invention, the formulation of Ar₂ in cyano-derivatized oligonophenylacetylene based fluorescent small molecule formulation is as follows:

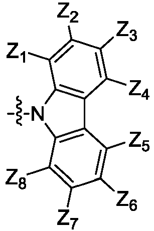

In the preferred application of the invention, Z₁-Z₈ in the Ar₂ formulation in the general formulation of the cyano-derivatized oligonophenylacetylene based fluorescent small molecule formulation is selected from hydrogen independently of one another or straight chain containing 1 to 40 carbons or branched saturated alkyl or alkoxy group or straight chain having alkene or alkyne functional groups or a group containing the aromatic amine group of the branched unsaturated alkyl or alkoxy group.

In the preferred application of the invention, a in cyano-derivatized oligonophenylacetylene based fluorescent small molecule formulation is any number between 0 and 3, b is 0 or 1.

Production method of cyano-derivatized oligonophenylacetylene based fluorescent small molecule subject of invention (100) includes the steps of
- X₁, X₂, Y₁, Y₂, Y₃, Y₄, Ar₁ and Ar₂ in general formulation is chemical compounds,
- a and b in the general formula are integers.

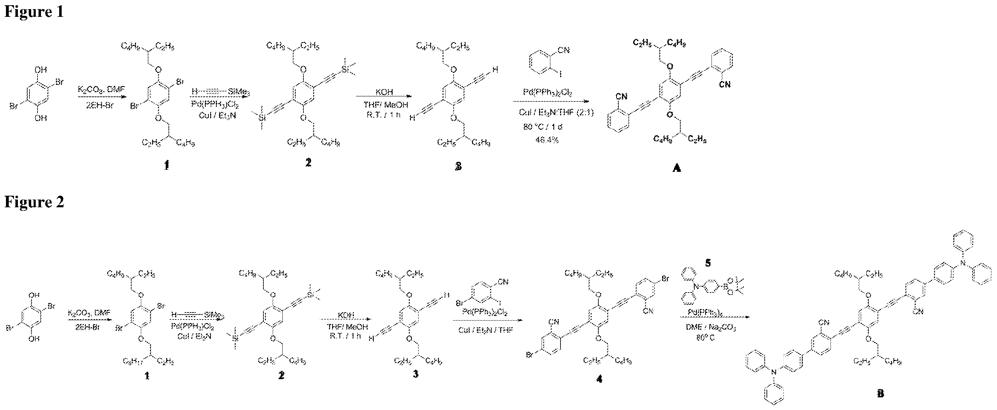

In one application of the invention, the general formulation of cyano-derivatized oligonophenylacetylene based fluorescent small molecule is as follows:
- X₁ and X₂ -O(CH₂)CH(C₂H₅)(C₄H₉) alkoxy group,
- Y₁ cyano (-CN) group,
- Y₂, Y₃ and Y₄ H atom,
- where a and b = 0, which yields small molecule ***2,2'-((2,5-bis((2-ethylhexyl)oxy)-1,4-phenylene)bis(ethyne-2,1-diyl))dibenzonitrile (A) (******Figure 1******)***.

In one application of the invention, taking advantage of the general formulation of cyano-derivatized oligonophenylacetylene based fluorescent small molecule, **2,2'-((2,5-bis((2-ethylhexyl)oxy)-1,4-phenylene)bis(ethyne-2,1-diyl))dibenzonitrile (A)** is synthesized (Figure 1). To carry out the synthesis, 1,4-dibromo-2,5-bis((2-ethylhexyl)oxy)benzene ***(1)*** is first synthesized. 2,5-dibromohydroquinone (1.455 g, 5.43 mmol) is slowly added to potassium carbonate (K₂CO₃) (1.5 g, 10.86 mmol) and 2-ethylhexyl bromide (2EH-Br) dissolved in dry DMF (dimethylformamide), and the mixture is stirred at 100 °C for 24 hours. The reaction mixture is cooled down to room temperature, poured into water, and the product is extracted with dichloromethane. The organic phase is dried with Na₂SO₄, filtered, and evaporated to dryness. The product is purified by flash chromatography using silica gel and hexane. The pure product is obtained as a colorless oil. The resulting 1,4-dibromo-2,5-bis((2-ethylhexyl)oxy)benzene (2.0 g, 4.06 mmol) ***(1)***, Pd(PPh₃)Cl₂ (0.1.71 g, 0.243 mmol), CuI (0.039 g, 0.203 mmol), Et₃N (triethylamine) (40 mL) are combined and stirred for 5 minutes. ethynyltrimethylsilane (1.0 g, 10.16 mmol) is added to the mixture and stirred at 90 °C for 24 hours. Then, the reaction was brought to room temperature and filtered. The solvent is completely evaporated, and the product is purified by flash chromatography using a mixture of silica gel and hexane: ethylacetate (30:1). ((2,5-bis((2-ethylhexyl)oxy)-1,4-phenylene)bis(ethyne-2,1-diyl))bis(trimethylsilane) ***(2)*** is obtained as a yellow oil. The resulting ((2,5-bis((2-ethylhexyl)oxy)-1,4-phenylene)bis(ethyne-2,1-diyl))bis(trimethylsilane) ***(2)*** (1.45 g, 2.75 mmol) and KOH ( 4.65 g, 82.55 mmol) reagents are stirred in THF: methanol (MeOH) (9: 1) (150 mL) for 1 hour. The mixture is poured into water and the product is extracted with dichloromethane. The organic phase is dried with Na₂SO₄, filtered, and the solvent is evaporated. The product is purified by flash chromatography using silica gel and hexane. 1,4-bis((2-ethylhexyl)oxy)-2,5-diethynylbenzene ***(3)*** is obtained as a yellow solid-oil. 2- iodobenzonitrile (0.514 g, 2.24 mmol) is then stirred in solution of CuI(0.0097g, 0.051 mmol) and Pd(PPh₃)₂Cl₂ (0.071 g 0.102 mmol) Et₃N:THF (2:1) (21 mL) for 5 minutes. 1,4-bis((2-ethylhexyl)oxy)-2,5-diethynylbenzene ***(3)*** (0.391 g, 1.02 mmol) dissolved in 5 mL of THF is slowly added and stirred at 80 °C for 24 hours. The reaction mixture is cooled down to room temperature. The solvent is completely evaporated, and the product is purified by flash column chromatography using silica gel and a mixture of dichloromethane:hexane (5:1). ***2,2'-((2,5-bis((2-ethylhexyl)oxy)-1,4-phenylene)bis(ethyne-2,1-diyl))dibenzonitrile (A)* is obtained** as a yellow solid.

In one application of the invention, where the formulation of cyano-derivatized oligonophenylacetylene based fluorescent small molecule is as follows:
- X₁ and X₂ -O(CH₂)CH(C₂H₅)(C₄H₉) alkoxy group,
- Y₁ cyano (-CN) group,
- Y₂, Y₃ and Y₄ H atom,
- a and b = 1,
- Ar₁ "benzene" aryl group,
- Ar₂ aromatic amine group 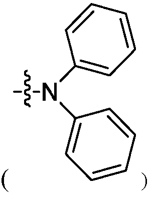 which yields small molecule ***4,4"-((2,5-bis((2-ethylhexyl)oxy)-1,4-phenylene)bis(ethyne-2,1-diyl))bis(4'-(diphenylamino)-[1,1'-biphenyl]-3-carbonitrile) (B) (******Figure 2******).***

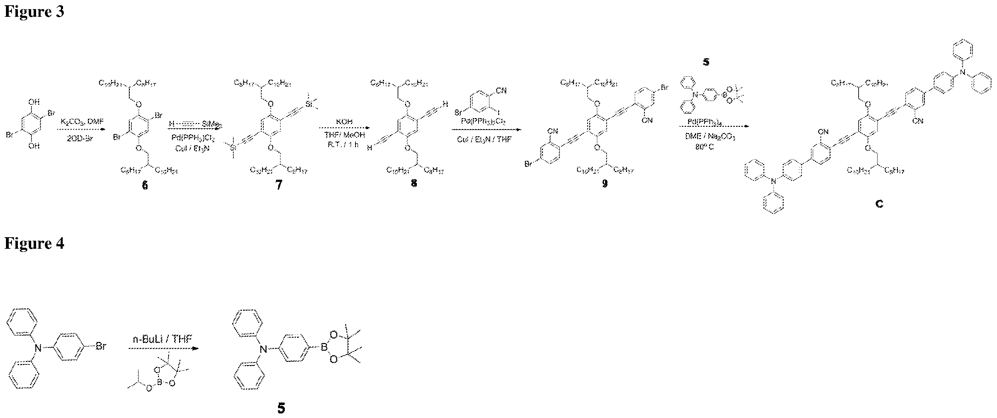

In one application of the invention, taking advantage of the general formulation of cyano-derivatized oligonophenylacetylene based fluorescent small molecule, ***4,4"-((2,5-bis((2-ethylhexyl)oxy)-1,4-phenylene)bis(ethyne-2,1-diyl))bis(4'-(diphenylamino)-[1,1'-biphenyl]-3-carbonitrile) (B)*** is synthesized (Figure 2). In this synthesis, 5-bromo-2-iodobenzonitrile (1.64 g, 5.36 mmol), CuI(0.02 g, 0.108 mmol) and Pd(PPh₃)₂Cl₂ (0.152 g 0.054 mmol) is first stirred in Et₃N:THF (2:1) (48 mL) for 5 minutes. **1,4-bis((2-ethylhexyl)oxy)-2,5-diethynylbenzene *(3)*** (0.82 g, 2.14 mmol)) dissolved in 12 mL THF is slowly added and stirred at 80 °C for 24 hours. The reaction mixture is cooled to room temperature. The solvent is completely evaporated and the product is purified by flash chromatography using silica gel and a mixture of hexane: ethylacetate (3: 1). Thus 6,6'-((2,5-bis((2-ethylhexyl)oxy)-1,4-phenylene)bis(ethyne-2,1-diyl))bis(3-bromobenzonitrile) ***(4)*** is obtained as yellow solid. 4-bromotriphenylamine (1.0 g, 3.08 mmol) dissolved in 40 mL of THF is then cooled to -78 ° C and 1.36 mL (3.4 mmol) of n-butyllithium (2.5 M n-hexane) are added dropwise thereto. After the mixture was stirred at -78 °C for 1 hour, 2-Isopropoxy-4,4,5,5-tetramethyl-1,3,2-dioxaborolane (0.688 g, 3.7 mmol) is slowly added at -78 ° C and the reaction is slowly warmed to room temperature and stirred overnight. The mixture is poured into water and the product is extracted with chloroform. The organic phase is dried with Na₂SO₄, filtered and the solvent is evaporated completely.N,N-diphenyl-4-(4,4,5,5-tetramethyl-1,3,2-dioxaborolan-2-yl) aniline ***(5)*** is obtained as pink solid (Synthesis is shown in Figure 4.). 6,6'-((2,5-bis((2-ethylhexyl)oxy)-1,4-phenylene)bis(ethyne-2,1-diyl))bis(3-bromobenzonitrile) ***(4)*** (1.04 g, 1.4 mmol) dissolved in dimethoxyethane (60 mL) is mixed with N,N-diphenyl-4-(4,4,5,5-tetramethyl-1,3,2-dioxaborolan-2-yl) aniline ***(5)*** (1.16 g, 3.08 mmol), Pd(PPh₃)₄ (0.08 g, 0.07 mmol) and Na₂CO₃ (1.304 g, 12.32 mmol) dissolved in 8 mL of deionized water is slowly added and stirred at 80 °C for 24 hours. The reaction mixture is cooled to room temperature. The solvent is completely evaporated and the product is purified by flash chromatography using silica gel and a mixture of dichloromethane:hexane (3:1). ***4,4"-((2,5-bis((2-ethylhexyl)oxy)-1,4-phenylene)bis(ethyne-2,1-diyl))bis(4'-(diphenylamino)-[1,1'-biphenyl]-3-carbonitrile) (B)*** is obtained as a yellow solid.

In one application of the invention, the general formulation of cyano-derivatized oligonophenylacetylene based fluorescent small molecule is as follows:
- X₁ and X₂ -O(CH₂)CH(C₈H₁₇)(C₁₀H₂₁) alkoxy group,
- Y₁ cyano (-CN) group,
- Y₂, Y₃ and Y₄ H atom,
- a and b = 1,
- Ar₁ "benzene" aryl group,
- Ar₂ aromatic amine group 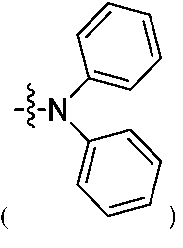 which yields small molecule ***4,4"-((2,5-bis((2-octyldodecyl)oxy)-1,4-phenylene)bis(ethyne-2,1-diyl))bis(4'-(diphenylamino)-[1,1'-biphenyl]-3-carbonitrile) (C) (******Figure 3******).***

In one application of the invention, taking advantage of the general formulation of cyano-derivatized oligonophenylacetylene based fluorescent small molecule, **4,4"-((2,5-bis((2-octyldodecyl)oxy)-1,4-phenylene)bis(ethyne-2,1-diyl))bis(4'-(diphenylamino)-[1,1'-biphenyl]-3-carbonitrile) (C)** is synthesized (Figure 3). To achieve the synthesis, 2,5-dibromohydroquinone (2.06 g, 7.47 mmol) was first added to potassium carbonate (K₂CO₃) (2.063 g, 14.93 mmol) dissolved in dry DMF and 2-octyldodecyl bromide (2OD-Br) (7.55 g, 20.9 mmol) is slowly added, and the mixture is stirred at 100 ° C for 48 hours. The reaction mixture is cooled to room temperature and poured into water, and the product is extracted with dichloromethane. The organic phase is dried with Na₂SO₄, filtered and the solvent is evaporated. The product is purified by flash chromatography using silica gel and hexane. Thus, 1,4-dibromo-2,5-bis((2-octyldodecyl)oxy)benzene ***(6)*** is obtained as colorless oil. 1,4-dibromo-2,5-bis((2-octyldodecyl)oxy)benzene ***(6)*** (3.0 g, 3.62 mmol), Pd(PPh₃)₂Cl₂ (0.152 g, 0.217 mmol), CuI (0.035 g, 0.181 mmol), Et₃N (60 mL) are combined and stirred for 5 minutes. Ethynyltrimethylsilane (0.889 g, 9.05 mmol) is added to the mixture and stirred at 90 ° C for 24 hours. The reaction is then brought to room temperature and filtered. The solvent is completely evaporated and the product is purified by flash chromatography using a mixture of silica gel and hexane:ethylacetate (30:1). ((2,5-bis((2-octyldodecyl)oxy)-1,4-phenylene)bis(ethyne-2,1-diyl))bis(trimethylsilane) ***(7)*** is obtained as a yellow oil. ((2,5-bis((2-octyldodecyl)oxy)-1,4-phenylene)bis(ethyne-2,1-diyl))bis(trimethylsilane) ***(7)*** (1.0 g, 1.158 mmol) and KOH ( 1.95 g, 34.74 mmol) reagents are stirred in THF:methanol (9:1) (60 mL) for 1 hour. The mixture is poured into water and the product is extracted with dichloromethane. The organic phase is dried with Na₂SO₄, filtered and the solvent is evaporated. The product is purified by flash chromatography using silica gel and hexane. 1,4-bis((2-octyldodecyl)oxy)-2,5-diethynylbenzene ***(8)*** is obtained as yellow solid-oil. 5-bromo-2-iodobenzonitrile (0.788 g, 2.56 mmol), CuI (0.011 g, 0.059 mmol) and Pd(PPh₃)₂Cl₂ (0.084 g 0.1195 mmol) are then stirred in mixture of Et₃N:THF (2:1) (33 mL) for 5 minutes. 1,4-bis((2-octyldodecyl)oxy)-2,5-diethynylbenzene (0.80 g, 1.11 mmol) dissolved in 3 mL THF is slowly added and stirred at 80 °C for 24 hours. The reaction mixture is cooled down to room temperature. The remained solvent is completely evaporated and the product is purified by flash chromatography using a mixture of silica gel and hexane: ethylacetate (10: 1). 6,6'-((2,5-bis((2-octyldodecyl)oxy)-1,4-phenylene)bis(ethyne-2,1-diyl))bis(3-bromobenzonitrile) ***(9)*** is obtained as yellow solid. 6,6'-((2,5-bis((2-octyldodecyl)oxy)-1,4-phenylene)bis(ethyne-2,1-diyl))bis(3-bromobenzonitrile) ***(9)*** (0.583 g, 0.54 mmol) dissolved in dimethoxyethane (15 mL), 1. N,N-diphenyl-4-(4,4,5,5-tetramethyl-1,3,2-dioxaborolan-2-yl) aniline ***(5)*** (0.431 g, 1.16 mmol), Pd(PPh₃)₄ (0.03 g, 0.027 mmol) are mixed and Na₂CO₃ (0.504 g, 4.75 mmol) dissolved in 3 mL deionized water is slowly added on it and stirred at 80 °C for 24 hours. The reaction mixture is cooled down to room temperature. The solvent in it is completely evaporated and the product is purified by flash chromatography using silica gel and a mixture of dichloromethane: hexane (1: 1). Thus ***4,4"-((2,5-bis((2-octyldodecyl)oxy)-1,4-phenylene)bis(ethyne-2,1-diyl))bis(4'-(diphenylamino)-[1,1'-biphenyl]-3-carbonitrile) (C)*** is obtained as a yellow solid.

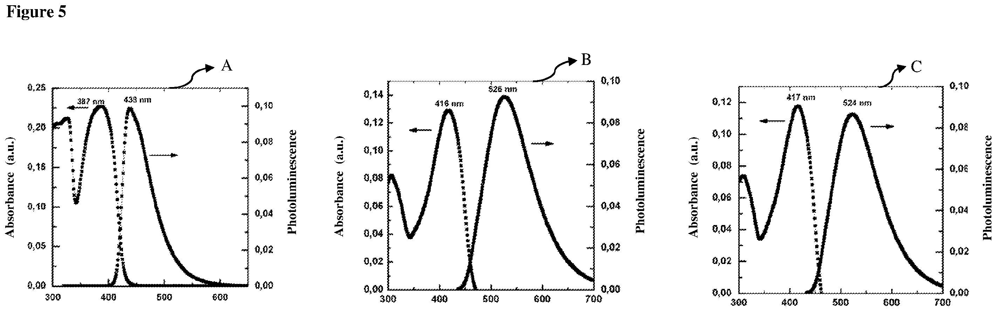

In one application of the invention, the cyano-derivatized oligonophenylacetylene-based small molecules (A, B, C) obtained as shown in Figure 5 exhibit absorbance and photoluminescence features at different wavelengths in their dichloromethane solutions.

The invention also relates to the use of synthesized cyano-derivatized oligonophenylacetylene-based fluorescent small molecules as the light-emitting active layer in OLED.

The cyano-derivatized oligonophenylacetylene-based small molecules obtained in one application of the invention exhibit electroluminescence in the OLED.

The invention also relates to a method of forming OLEDs comprising cyano-derivatized oligonophenylacetylene-based fluorescent small molecule layer.

A method of forming OLEDs comprising cyano-derivatized oligonophenylacetylene based fluorescent small molecule layer subject of invention includes the steps of
- washing coated glass substrates (D) of ITO (indium tin oxide) (E) used as basic material in OLED,
- coating ITO (indium tin oxide) (E) coated glass substrate (D) surface with PEDOT:PSS (poly-ethylenedioxythiophene: polystyrenesulfonate) (F),
- coating light-emitting small molecules on PEDOT:PSS (poly-ethylenedioxythiophene: polystyrenesulfonate) (F) layer,
- coating light-emitting small molecular layer with TPBI (I) as the electron-transport layer,
- depositing cathode and electron injection material of Al (K) / LiF (L) or Al (K) / Ca (J).

In the method of forming the OLEDs subject of invention, the ITO (indium tin oxide) (E) coated 1x1 inch square glass substrates are washed in ultrasonic bath with detergent water, purified water, acetone, ethanol and isopropanol for 10 minutes respectively. After washing, the substrates are dried with nitrogen (N₂) gas and cured in ozone cleaner for 5 minutes. PEDOT:PSS (poly-ethylenedioxythiophene: polystyrenesulfonate) (F) solution is filtered and coated on the cleaned glass substrate by spin-coating. Heat treatment is then carried out at 120 °C for 20 minutes. Solutions of small molecules (A, B, C - 2) in chloroform are prepared. In some cases, the host material (CBP) (A, B, C - 1) can be added while the solution is being prepared. Using a solution prepared from light-emitting small molecules, the glass substrate (D) is coated with the spin-coating device. Annealing is carried out at 80°C for 30 minutes after coating. The electron transport material (TPBI) (I) on the light-emitting layer is vacuum coated at 350 °C and finally the cathode material 10 nm Ca (J) / 100 nm Al (K) or 1 nm LiF (L) / 100 nm Al (K) is coated by thermal evaporation method.

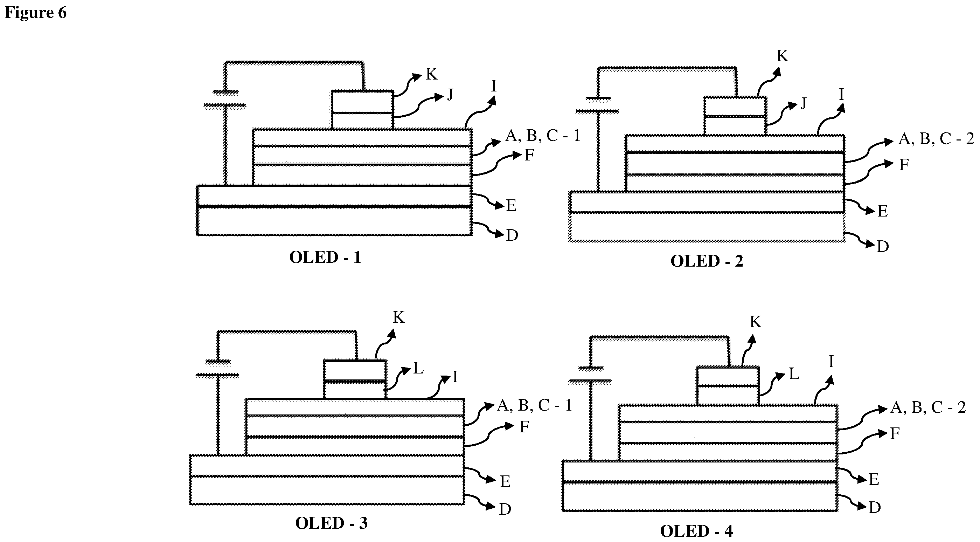

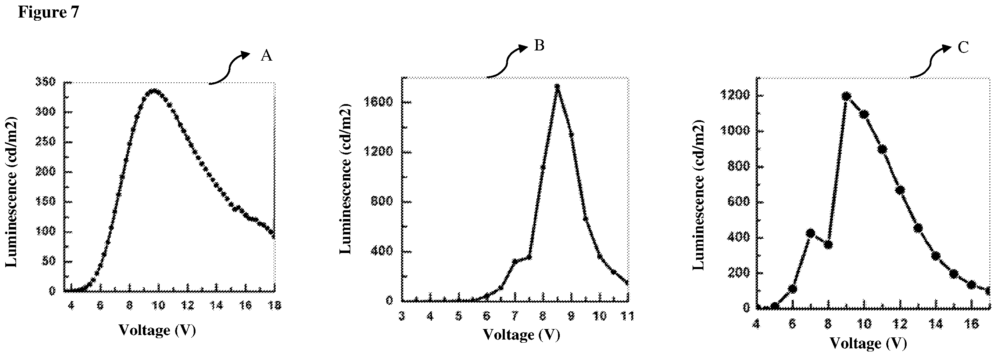

Figure 6 shows four different structures of small molecules used in OLED testing. OLED No. 1 having the small molecule to be tested contains respectively; ITO (E), PEDOT:PSS (F), CBP:small molecule (A, B, C - 1), TPBI (I), Ca (J) and Al (K). OLED No. 2 contains respectively; ITO (E), PEDOT:PSS (F), small molecule (A, B, C - 2), TPBI (I), Ca (J) and Al (K). Tested OLED No. 3 contains respectively; ITO (E), PEDOT:PSS (F), CBP:small molecule (A, B, C - 1), TPBI (I), LiF (L) and Al (K); OLED No. 4 contains respectively; ITO (E), PEDOT:PSS (F), small molecule (A, B, C - 2), TPBI (I), LiF (L) and Al (K). EL, glare, EQE, current output, current density-voltage data and color coordinates of the OLEDs tested are measured. Table 1 gives the electrical characterization data of OLED devices containing cyano-derivatized oligophenylacetylene molecules (A, B and C). The glare graphics of the respective devices are also shown in Figure 7.

**Table 1. Electrical characterization data of OLED devices containing cyano-derivatized oligophenylacetylene molecules (A, B and C)**

| Name of small molecule | Stracture of OLED device | Glare Lₘₐₓ[cd/m²] | Brightness Yield LEₘₐₓ[cd/A] | External Quantum Yield EQE[%] | EL λmax[nm] | CIE 1931 (X, Y) |
|---|---|---|---|---|---|---|
| Cyano-oligophenylacetylene-1 | 1 | 335 | 5.9 | 6.97 | 452 | 0.15,0.10 |
| Cyano-oligophenylacetylene-2 | 3 | 1227 | 10 | 5.50 | 473 | 0.17,0.25 |
| Cyano-oligophenylacetylene-2 | 4 | 1730 | 12 | 4.3 | 510 | 0.29,0.49 |
| Cyano-oligophenylacetylene-3 | 1 | 1196 | 10.5 | 4.67 | 476 | 0.19,0.34 |
| Cyano-oligophenylacetylene-3 | 2 | 1434 | 5.15 | 1.78 | 554 | 0.40,0.53 |

In a preferred application of the invention, the resulting cyano-derivatized oligonophenylacetylene-based small molecules are used in a light-emitting layer structure in OLED applications by means of a coating process at low cost, resulting in high luminescence, external quantum efficiency, and radiation efficiency.

Based on these basic concepts, it is possible to develop a variety of applications for cyano-derivatized oligonophenylacetylene-based fluorescent small molecules and their use in organic light-emitting diode applications suitable for use in organic light-emitting diode applications, the invention not being limited to the examples described herein, it is exactly as specified in claims.

## Claims

1. The cyano-derivatized oligonophenylacetylene-based fluorescent small molecule
***2,2'-((2,5-bis((2-ethylhexyl)oxy)-1,4-phenylene)bis(ethyne-2,1-diyl))dibenzonitrile (A)* characterized in that** in the general formula,

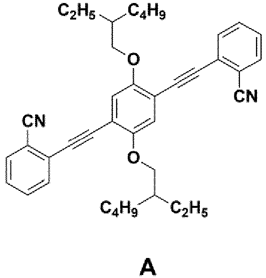

2. The cyano-derivatized oligonophenylacetylene-based fluorescent small molecule
***4,4"-((2,5-bis((2-ethylhexyl)oxy)-1,4-phenylene)bis(ethyne-2,1-diyl))bis(4'-(diphenylamino)-[1,1'-biphenyl]-3-carbonitrile) (B)* characterized in that** in the general formula,

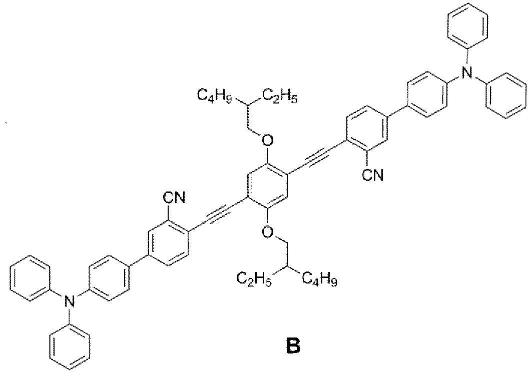

3. The cyano-derivatized oligonophenylacetylene-based fluorescent small molecules
***4,4''-((2,5-bis((2-octyldodecyl)oxy)-1,4-phenylene)bis(ethyne-2,1-diyl))bis(4'-(diphenylamino)-[1,1'-biphenyl]-3-carbonitrile) (C)* characterized in that** in the general formula,

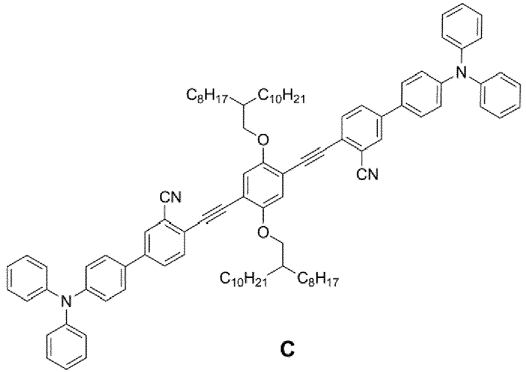

4. Using the cyano-derivatized oligonophenylacetylene-based fluorescent small molecules according to Claim 1 to Claim 3 as the light-emitting active layer in OLED devices.

## Patentansprüche

1. Das Cyano-derivatisierte fluoreszierende kleine Molekül auf der Basis von Oligonophenylacetylen ***2,2'-((2,5-Bis((2-ethylhexyl)oxy)-1,4-phenylen)bis(ethyn-2,1-diyl))dibenzonitril (A),* dadurch gekennzeichnet, dass** es die allgemeine Formel hat,

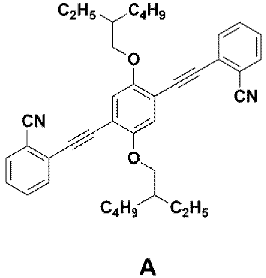

2. Das Cyano-derivatisierte fluoreszierende kleine Molekül auf der Basis von Oligonophenylacetylen ***4,4"-((2,5-Bis((2-ethylhexyl)oxy)-1,4-phenylen)bis(ethyn-2,1-diyl))bis(4'-(diphenylamino)-[1,1'-biphenyl]-3-carbonitril) (B),* dadurch gekennzeichnet, dass** es die allgemeine Formel hat,

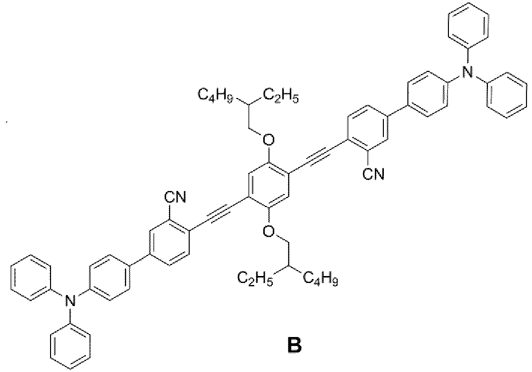

3. Das Cyano-derivatisierte fluoreszierende kleine Molekül auf der Basis von Oligonophenylacetylen;
***4,4"-((2,5-bis((2-octyldodecyl)oxy)-1,4-phenylen)bis(ethyn-2,1-diyl))bis(4'-(diphenylamino)-[1,1'-biphenyl]-3-carbonitril) (C)*, dadurch gekennzeichnet, dass** es die allgemeine Formel hat,

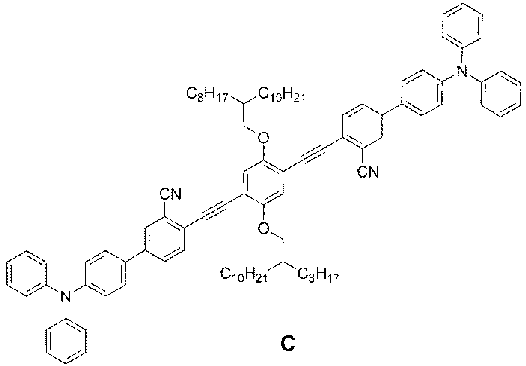

4. Verwendung von Cyano-derivatisierten fluoreszierenden kleinen Molekülen auf der Basis von Oligonophenylacetylen nach Anspruch 1 bis Anspruch 3 als lichtemittierende aktive Schicht in OLED-Vorrichtungen.

## Revendications

1. La petite molécule fluorescente à base d'oligonophénylacétylène cyano-dérivatisée
***2,2'-((2,5-bis((2-éthylhexyl)oxy)-1,4-phénylène)bis(éthyn-2,1-diyl))dibenzonitrile (A)* caractérisée en ce qu'**il présente de la formule générale,

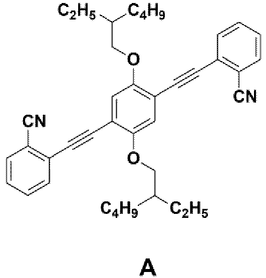

2. La petite molécule fluorescente à base d'oligonophénylacétylène cyano-dérivatisée
***4,4"-((2,5-bis((2-éthylhexyl)oxy)-1,4-phénylène)bis(éthyn-2,1-diyl))bis(4'-(diphenylamino)-[1,1'-biphényl]-3-carbonitrile) (B)* caractérisée en ce qu'**il présente de la formule générale,

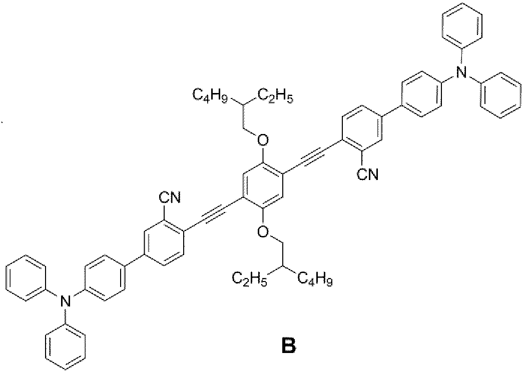

3. La petite molécule fluorescente à base d'oligonophénylacétylène cyano-dérivatisée
***4,4"-((2,5-bis((2-octyldodécyl)oxy)-1,4-phénylène)bis(éthyn-2,1-diyl))bis(4'-(diphénylamino)-[1,1'-biphényl]-3-carbonitrile) (C)* caractérisée en ce qu'**il présente de la formule générale,

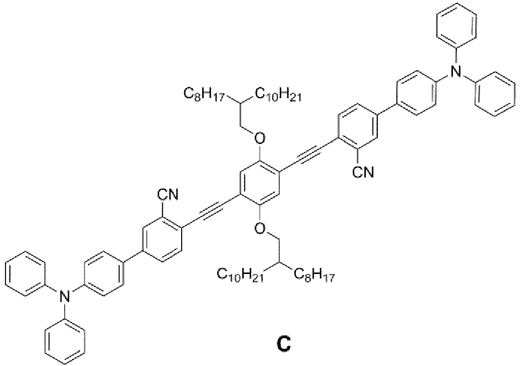

4. Utilisation des petites molécules fluorescentes à base d'oligonophénylacétylène cyano-dérivatisées selon la revendication 1 à la revendication 3 en tant que la couche active électroluminescente dans des dispositifs OLED.
